# EUROPÄISCHE PATENTSCHRIFT

(11) **EP 1 108 064 B1**
(45) Veröffentlichungstag und Bekanntmachung des Hinweises auf die Patenterteilung: **21.03.2007**
(21) Anmeldenummer: 99946095.9
(22) Anmeldetag: 02.09.1999
(51) Int. Cl.: C12Q 1/68

(54) **OLIGONUKLEOTIDE, VERFAHREN UND KIT ZUR DETEKTION VON LISTERIA MONOCYTOGENES DURCH NUKLEINSÄUREAMPLIFIKATION UND/ODER -HYBRIDISIERUNG**
OLIGO NUCLEOTIDES, METHOD AND KIT FOR DETECTING LISTERIA MONOCYTOGENES BY AMPLIFYING AND/OR HYBRIDIZING NUCLEIC ACIDS
OLIGONUCLEOTIDES, PROCEDE ET KIT DE DETECTION DU MONOCYTOGENE DE LA LISTERIA PAR AMPLIFICATION ET/OU HYBRIDATION D'ACIDE NUCLEIQUE

(30) Priorität: 02.09.1998 DE 1984004 B
(43) Veröffentlichungstag der Anmeldung: 20.06.2001
(73) Patentinhaber: Biotecon Diagnostics GmbH, 10589 Berlin (DE)
(72) Erfinder: SCHEU, Pia, D-12101 Berlin (DE); GASCH, Alexander, D-13359 Berlin (DE); BERGHOF, Kornelia, D-12355 Berlin (DE)
(74) Vertreter: Boeters, Hans Dietrich
(86) Internationale Anmeldenummer: PCT/EP1999/006453
(87) Internationale Veröffentlichungsnummer: WO 2000/014276

(56) Entgegenhaltungen:
- EP-A- 0 576 842
- WO-A-90/08841
- WO-A-92/01063
- WO-A-98/20160
- DATABASE EMBL [Online] ID: SPU97390 , Juli 1997 (1997-07) JANG: "SCHIZOSACCHAROMYCES POMBE 40S RIBOSOMAL PROTEIN S4 HOMOLOG mRNA PARTIAL CDS" XP002133546
- DATABASE GENESEQ [Online] ID/AC: T36015, April 1997 (1997-04) ARUFFO ET AL.: "ANTISENSE PRIMER FOR SIGNAL PEPTIDE REGION OF HUMANISED ANTIBODY" XP002133547
- DATABASE EMBL [Online] ID/AC: A42883, März 1997 (1997-03) ABKEN ET AL: "SEQUENCE 15 FROM WO9502701 "METHOD OF IDENTIFYING HUMAN AND ANIMAL CELLS CAPABLE OF UNLIMITED PROLIFERATION OR TUMOUR FORMATION"" XP002133548
- DATABASE GENESEQ [Online] ID/AC: T47423, September 1997 (1997-09) SHUBER: "PRIMER #4 FOR TAY-SACHS DISEASE GENE" XP002133549
- DATABASE EMBL [Online] ID/AC: AA657460, November 1997 (1997-11) EMMERT-BUCK: "Homo sapiens cDNA clone IMAGE:1203441" XP002133550
- DATABASE GENESEQ [Online] ID/AC: Q20004, Dezember 1991 (1991-12) MATTEUCCI: "TARGET DUPLEX FOR COVALENTLY CROSS-LINKING OLIGONUCLEOTIDES" XP002133551
- DOMANN ET AL.: "MOLECULAR CLONING, SEQUENCING AND IDENTIFICATION OF A METALLOPROTEASE GENE FROM LISTERIA MONOCYTOGENES THAT IS SPECIES SPECIFIC AND PHYSICALLY LINKED TO THE LISTERIOLYSIN GENE" INFECTION AND IMMUNITY, Bd. 59, Nr. 1, 1991, Seiten 65-72, XP002133545 in der Anmeldung erwähnt
- ROSSEN L ET AL: "A RAPID POLYMERASE CHAIN REACTION (PCR)-BASED ASSAY FOR THE IDENTIFICATION OF LISTERIA MONOCYTOGENES IN FOOD SAMPLES" INTERNATIONAL JOURNAL OF FOOD MICROBIOLOGY,NL,ELSEVIER SCIENCE PUBLISHERS, AMSTERDAM, Bd. 14, 1. Januar 1991 (1991-01-01), Seiten 145-151, XP000198173 ISSN: 0168-1605 in der Anmeldung erwähnt
- SCHEU ET AL.: "RAPID DETECTION OF LISTERIA MONOCYTOGENES BY PCR-ELISA" APPLIED MICROBIOLOGY, Bd. 29, Nr. 6, Dezember 1999 (1999-12), Seiten 416-420, XP000892485

## Beschreibung

Erfindungsgemäß werden Oligonukleotide, Verfahren und Kit zur Detektion *von Listeria monocytogenes* durch Nukleinsäureamplifikation und/oder -hybridisierung bereitgestellt.

Die Gattung *Listeria* besteht aus den sechs Spezies *L. monocytogenes, L. grayi, L. innocua*, *L. ivanovii, L. seligeri* und *L. welshimeri.* Unter diesen sind nur Stämme der Spezies *L*. *monocytogenes* pathogen für den Menschen, insbesondere für Immungeschwächte, Ältere und Neugeborene. Die häufigsten Symptome der Listeriose sind Septikämie, Meningitis und Fehlgeburten. Infektionen durch *L. monocytogenes* werden vor allem durch die Aufnahme kontaminierter Lebensmittel verursacht, insbesondere von Milchprodukten, Fleisch, Geflügel und Gemüse.

Eine Vielzahl von Verfahren zur Detektion von *L. monocytogenes* sind bekannt. Konventionelle Detektionsverfahren für *L. monocytogenes* bestehen aus einer Voranreicherung und nachfolgender Isolation von Kolonien auf Selektivmedien (Lovett et al., J. Food Protection **50** (1987), 188-192; McClain & Lee, J. Assoc. Off. Anal. Chem. **71** (1988), 660-664). Einzelkolonien werden nach ihrer Morphologie bzw. nach biochemischen oder serologischen Eigenschaften untersucht. Die Durchführung einer Analyse kann bis zu 6 - 8 Tage in Anspruch nehmen.

Da vor allem leicht verderbliche Nahrungsmittel häufig mit *L. monocytogenes* kontaminiert sind, wurden verschiedene Schnellverfahren zur Detektion von *L. monocytogenes* entwickelt. Diese Verfahren basieren entweder auf immunologischen Methoden oder der Anwendung von Nukleinsäuresonden.

Dabei kann die Detektion durch direkte Hybridisierung von Sonden an keimspezifische DNA bzw. RNA erfolgen (siehe z.B. Datta, A. R. et al., Appl. Environ. Microbiol. **53** (1987), 2256-2259). Der Nachteil bei solchen Verfahren ist die geringe Sensitivität, da mindestens 10⁵-10⁶ Kopien der Zielnukleinsäure erforderlich sind. Dies kann durch Kombination mit einer Vervielfältigung der Zielsequenz, z.B, durch die Polymerase-Kettenreaktion (PCR), ausgeglichen werden. Mehrere PCR-Verfahren zum Nachweis von *L. monocytogenes* sind in der Literatur beschrieben [zur Übersicht siehe z.B. Jones, D.D. & Bej, A.K. in "PCR Technology, Current Innovations", Griffin, H.G. & Griffin, A.M., Hrsg., (1994), 341-365]. Siehe auch die US Patente 4,683,195; 4,683,202 und 4,965,188. Ferner können die Ligase-Kettenreaktion [WO Veröffentlichung 89/09835], die "self-sustained sequence replication" [EP 329,822], das "transcription based amplification system" [EP 310,229] und das Qβ RNA-Replikase-System [US Patent 4,957,858] zur Amplifikation von Nukleinsäuren eingesetzt werden.

Es sind auch bereits einige Testkits zur Detektion mittels Antikörpern kommerziell erhältlich. Die meisten dieser Tests zeigen jedoch nur eine geringe Sensitivität und Spezifität.

Zur Detektion von spezifischen Mikroorganismen mittels Nukleinsäure-Hybridisierung oder -Amplifikation werden üblicherweise keimspezifische Oligonukleotide verwendet, deren Basensequenz für die DNA oder RNA eines spezifischen Mikroorganismus oder einer Gruppe von Mikroorganismen charakteristisch ist. Eine Hybridisierung an die DNA/RNA bzw. eine Amplifikation von DNA/RNA bei Einsatz dieser keimspezifischen Oligonukleotide (z.B. als Primer oder Sonden) mit den oben genannten Verfahren kann, unter geeigneten Reaktionsbedingungen, nur dann erfolgen, wenn die DNA/RNA der jeweils nachzuweisenden Mikroorganismen anwesend ist.

Die für *L. monocytogenes* beschriebenen Nachweisverfahren basieren überwiegend auf solchen Zielgenen, die eine Rolle in der Pathogenität von *L. monocytogenes* spielen. Es ist bekannt, daß einige dieser Gene in einem Virulenz-Gencluster benachbart auf dem Chromosom angeordnet sind. Da das Listeriolysin-Gen (hlyA) zuerst als eindeutig notwendig für die Pathogenität von *L. monocytogenes* erkannt wurde (Cossart, P. et al., Infect. Immun. **57** (1989), 3629-3636), basieren die meisten genotypischen Nachweisverfahren auf diesem Gen. Das hlyA-Gen kommt jedoch mit hoher Homologie auch bei apathogenen Listerien vor (i.e. bei *L. seeligeri* und *L. ivanovii*). Das Auftreten von falsch-positiven Ergebnissen ist bei diesen Nachweisverfahren nicht mit vollständiger Sicherheit auszuschließen, da einzelne Punktmutationen im Bereich der Bindungsstellen von Primern oder Sonden hierzu bereits ausreichen können.

Für das dem hlyA-Gen im Genom direkt benachbarte Metalloprotease-Gen (mpl) konnte gezeigt werden, daß es nur bei *L. monocytogenes* vorkommt, also nicht bei apathogenen Listerien (Domann, E. et al., Infect. Immun. **59** (1991), 65-72).

Die prinzipielle Eignung der das hlyA-Gen flankierenden DNA-Region zum Nachweis von *L. monocytogenes* mittels Hybridisierung oder Amplifikation ist beschrieben (Rossen, L. et al., Int. J. Food Microbiol. **14** (1991), 145-152), jedoch sind bisher für solche Nachweisverfahren keine Oligonukleotidsequenzen publiziert worden.

Die Sequenz des mpl-Gens von *L. monocytogenes* ist in der EMBL Datenbank unter der Accession Nummer X54619 beschrieben [Domann, E. et al., Infect. Immun. **59** (1991), 65-72]. Desweiteren sind Teile der Sequenz des mpl-Gens von *L. monocytogenes* in der EMBL Datenbank unter der Accession Nummer X60035 aufgeführt [Rasmussen, O. F. et al., Infect. Immun. **59** (1991), 3945-3951].

Aufgabe der vorliegenden Erfindung war es nun, ein routine-geeignetes Nachweisverfahren zu entwickeln, bei dem auch unter stark schwankenden Versuchsbedingungen beim jeweiligen Anwender die Wahrscheinlichkeit für das Auftreten falsch-positiver Ergebnisse möglichst gering ist.

Insbesondere sollen Oligonukleotidsequenzen bereitgestellt werden, die in einem Nachweisverfahren für das Metallprotease-Gen (mpl) von L. monocytogenes eingesetzt werden können.

Diese Aufgaben werden durch die Bereitstellung von Nukleinsäuremolekülen der Sequenzen
(i) 5'-GAA AAA GCA TTT GAA GCC AT-3' oder
(ii) 5'-GCA ACT TCC GGC TCA GC-3' oder
(iii) 5'-TCG AAA AAG CAT TTG AAG CC-3' oder
(iv) 5'-GGT CAG AGT GAA GCT CAT GT-3' oder
(v) 5'-CTI TTC ACA TGA GCT TCA CTC TGA CCR A-3' oder
(vi) 5'-CTT TTT CTT TCA CTG GGT TTC CGA CAT-3' oder
(vii) 5'-GAT GAT TTC TTT TTC TTT CAC TGG ATT TCC AAT AT-3' oder
(viii) der zu (i), (ii), (iii), (iv), (v), (vi) und (vii) jeweils komplementären Sequenz gelöst.

Die erfindungsgemäßen Oligonukleotide können wie folgt definiert sein:

oligonukleotid LM1: (sequenz (i) = SEQ ID NO 1 entspricht der Position 2476 bis 2495 von *L. monocytogenes* [nach Domann, E. et al. Infect. Immun. **59** (1991), 65-72].
Oligonukleotid LM 2: (Sequenz (ii) = SEQ ID NO 2) entspricht der Position 2608 bis 2624 von *L. monocytogenes.* Oligonukleotid LM 3: (Sequenz (iii) = SEQ ID NO 3) entsprichtder Position 2474 bis 2493 von *L. monocytogenes.* Oligonukleotid LM 4 (Sequenz (iv) = SEQ ID NO 4) entspricht der Position 2497 bis 2516 von *L. monocytogenes.*
Oligonukleotid LMR 1: (Sequenz (v) = SEQ ID NO 5) entspricht der Position 2495 bis 2522 von *L. monocytogenes.* Oligonukleotid LMF 1: (Sequenz (vi) - SEQ ID NO 6) entspricht der Position 2525 bis 2551 von *L. monocytogenes.*
Oligonukleotid LMF 2 (Sequenz (vii) = SEQ ID NO 7) entspricht der Position 2525 bis 2559 von *L. monocytogenes.*

Zur Untersuchung, inwieweit Sequenzvariationen des mpl-Gens innerhalb der Spezies *L. monocytogenes* auftreten, wurde ein internes Fragment von 300 Basenpaaren von 13 *L. monocytogenes* Stämmen verschiedener Serovare (2 Stämme der Serovare 1/2a, 1 Stamm des Serovars 1/2b, 1 Stamm des Serovars 1/2c, 1 Stamm des Serovars 3a, 1 Stamm des Serovars 3b, 1 Stamm des Serovars 3c, 1 Stamm des Serovars 4a, 1 Stamm des Serovars 4a/b, 1 Stamm des Serovars 4b, 1 Stamm des Serovars 4c, 1 Stamm des Serovars 4d und 1 Stamm des Serovars 7) sequenziert. Anhand der Sequenzvergleiche wurde überraschenderweise gefunden, daß die Oligonukleotide LM1, LM 2, LM3, LM 4, LMF 1, LMF 2, LMR 1 sowie dazu komplementäre Sequenzen in Nachweisverfahren für *L. monocytogenes* zu hoch-spezifischen Nachweisen führen. Dabei werden die Oligonukleotide LM4, LMR1, LMF1 und LMF2 bzw. die dazu komplementären Sequenzen vorzugsweise als Sonden verwendet.

Nukleinsäuremoleküle werden insbesondere bereitgestellt, die dadurch gekennzeichnet sind, daß sie bezüglich mindestens 10 aufeinanderfolgenden Nukleotiden ihrer Nukleotidkette
(a) mit 10 aufeinanderfolgenden Nukleotiden der vorstehenden Nukleinsäuremoleküle (i) bis (viii) identisch sind oder
(b) mit 9 von 10 aufeinanderfolgenden Nukleotiden der vorstehenden Nukleinsäuremoleküle (i) bis (viii) übereinstimmen oder
(c) mit 8 von 10 aufeinanderfolgenden Nukleotiden der vorstehenden Nukleinsäuremoleküle (i) bis (viii) übereinstimmen oder
(d) zu mindestens 90 % mit einem Nukleinsäuremolekül gemäß Anspruch 1 homolog sind.

Die Oligonucleotide können eine für Sonden oder Primer übliche Länge aufweisen, insbesondere für eine PCR-Reaktion, sie können ferner eine durch Amplifikation, insbesondere durch eine PCR-Reaktion, herstellbare Länge aufweisen, und vorzugsweise können sie 10 bis 250 Basen und insbesondere 15 bis 30 Basen lang sein.

Sie können einzelsträngig oder doppelsträngig vorliegen.

Geeignet als keimspezifische Oligonukleotide zum Nachweis von *L. monocytogenes* sind also Nukleinsäuren, vorzugsweise 10 bis 250 Basen und insbesondere 15 bis 30 Basen lang, die mindestens in einer 10 Basen langen Sequenz mit den angegebenen Sequenzen LM 1, LM 2, LM 3, LM 4, LMF 1, LMF 2 und LMR 1 oder den hierzu komplementären Sequenzen übereinstimmen. Geringere Abweichungen (1 bis 2 Basen) in dieser 10 Basen langen Sequenz sind möglich, ohne daß die jeweils angegebene Spezifität bei der Amplifikation und/oder Hybridisierung verloren geht. Dem Fachmann ist bekannt, daß im Falle solcher geringeren Abweichungen die Reaktionsbedingungen entsprechend verändert werden müssen; vgl. beispielsweise T. Maniatis, Molecular Cloning, Herausgeber G. Sambrook & E.F. Fritsch, Cold Spring Harbour Laboratory Press, 1989.

Zum Nachweis von *L. monocytogenes* werden Nukleinsäuren, vorzugsweise genomische DNA, zunächst aus den in einer zu untersuchenden Probe bzw. Bakterienkultur enthaltenen Zellen freigesetzt. Mittels Nukleinsäure-Hybridisierung kann dann, und zwar unter Einsatz der erfindungsgemäßen keimspezifischen Oligonukleotide als Sonde, der direkte Nachweis von keimspezifischen Nukleinsäuresequenzen in der zu untersuchenden Probe erfolgen. Geeignet hierzu sind verschiedene dem Fachmann bekannte Verfahren, wie z.B., "Southern blot" oder "dot blot".

Bevorzugt ist jedoch, vor allem wegen der höheren Empfindlichkeit, ein indirektes Nachweisverfahren, bei dem die gesuchten und wie vorstehend beschriebenen freigesetzten DNA/RNA-Sequenzen zunächst mittels der o.g. Verfahren zur Amplifikation von Nukleinsäuren, vorzugsweise PCR, amplifiziert werden.

Die Amplifikation von DNA/RNA unter Verwendung der genannten Verfahren erfolgt unter Einsatz der erfindungsgemäßen Nukleinsäuremoleküle als Primer. Dabei werden nur in dem Fall spezifische Amplifikate gebildet, in dem DNA/RNA von *L. monocytogenes* anwesend ist. Durch eine Detektionsreaktion (nachgeschaltet bzw. während der Amplifikations-Reaktion) unter Verwendung der erfindungsgemäßen Nukleinsäuremoleküle als Sonden kann die Spezifität des Nachweisverfahrens erhöht werden. Für diese Detektionsreaktion ist ebenso die Verwendung von nicht vollständig keimspezifischen Oligonukleotiden möglich.

Alternativ kann die Nukleinsäure-Amplifikation auch in Anwesenheit eines oder mehrerer nicht vollständig spezifischer Oligonukleotide durchgeführt werden, so daß möglicherweise auch DNA/RNA anderer, nicht-nachzuweisender Mikroorganismen amplifiziert werden kann. Ein derartiges Amplifikationsverfahren ist in der Regel weniger spezifisch und sollte daher durch eine Detektionsreaktion (nachgeschaltet oder während der Amplifikations-Reaktion) mit einem oder mehreren der erfindungsgemäßen Nukleinsäuremolekül(e) als Sonde(n) abgesichert werden.

Erfindungsgemäß können verschiedene Verfahren eingesetzt werden, um die bei den indirekten Verfahren entstehenden Amplifikationsprodukte nachzuweisen. Dazu gehören u.a. an sich bekannte Verfahren wie die Visualisierung mittels Gelelektrophorese, die Hybridisierung von Sonden an immobilisierte Reaktionsprodukte [gekoppelt an Nylon-oder Nitrocellulose-Filter ("Southern blote") oder z.B. an "beads" oder Mikrotiterplatten] und die Hybridisierung der Reaktionsprodukte an immobilisierte Sonden (z.B. "reverse dot blots" oder mit Sonden gekoppelte "beads" oder Mikrotiterplatten). Zudem können Verfahren eingesetzt werden, bei dem ein oder mehrere erfindungsgemäße Nukleinsäuremoleküle als Sonden im Verlauf der PCR-Reaktion ("online") spezifisch entstehende Amplifikationsprodukte qualitativ und quantitativ nachweisen können.

Erfindungsgemäß gibt es eine Vielzahl von Möglichkeiten, mit denen die erfindungsgemäßen Oligonukleotide (z.B. Sonden und Primer) für die beschriebenen direkten oder indirekten Nachweisverfahren markiert bzw. modifiziert werden können. So können diese beispielsweise radioaktive, farbige, fluoreszierende oder anderweitig modifizierte bzw. modifizierende Gruppen enthalten, beispielsweise Antikörper, Antigene, Enzyme bzw. andere Substanzen mit Affinität zu Enzymen oder Enzymkomplexen. Sonden bzw. Primer können entweder natürlich vorkommende oder synthetisch hergestellte doppelsträngige oder einzelsträngige DNA oder RNA bzw. modifizierte Formen von DNA oder RNA, wie z.B. PNA sein (bei diesen Molekülen sind die Zucker-Einheiten durch Aminosäuren oder Peptide ausgetauscht). Einzelne oder mehrere Nukleotide der erfindungsgemäßen Sonden oder Primer können gegen analoge Bausteine (wie z.B. Nukleotide, die in der ZielNukleinsäure nicht natürlich vorkommen) ersetzt sein. Insbesondere können bis zu 20 % von mindestens 10 aufeinanderfolgenden Nukleotiden einer Nukleotidkette, insbesondere 1 oder 2 Nukleotide, durch für Sonden und/oder Primer an sich bekannte analoge Bausteine ersetzt werden.

Bei den o.g. indirekten Nachweisverfahren kann Detektion auch über ein intern-markiertes Amplifikat geführt werden. Dies kann z.B. über den Einbau von modifizierten (z.B. an Digoxigenin oder an Fluorescein gekoppelten) Nukleosidtriphosphaten während der Amplifikationsreaktion erfolgen.

Weiter wird erfindungsgemäß ein Kit für analytische Nachweisverfahren, insbesondere zum Nachweis von Bakterien der Spezies *Listeria monocytogenes,* bereitgestellt, der ein oder mehrere erfindungsgemäße Nukleinsäuremoleküle enthält.

Die erfindungsgemäßen Nukleinsäuremoleküle bzw. die entsprechenden Kits können in einem Verfahren zum Nachweis der An- oder Abwesenheit von Bakterien der Spezies *L. monocytogenes* in einer Probe verwendet werden, wobei es sich bei dem Verfahren vorzugsweise um eine Nukleinsäurehybridisierung und/oder eine Nukleinsäureamplifikation, wie eine PCR, handelt.

Dabei können nachzuweisende Bakterien von nicht-nachzuweisenden Bakterien anhand von Unterschieden der genomischen DNA und/oder RNA an mindestens einer Nukleotidposition im Bereich eines der erfindungsgemäßen Nukleinsäuremoleküle unterschieden werden.

### Beispiel

### Beispiel 1: Nachweis von Bakterien der Spezies L. monocytogenes mit der Polymerase-Kettenreaktion

Aus Reinkulturen der in Tabelle 1 aufgeführten Bakterien wurde DNA mittels Standardverfahren isoliert. Je ca. 10 bis 100 ng dieser DNA-Präparationen wurde dann in Gegenwart von je 0,4 µM Oligonukleotid LM 1 und LM 2, oder LM 3 und LM 2, 200 µM dNTP's (Boehringer Mannheim), 2,5 mM MgCl₂, 16 mM (NH₄)₂SO₄, 67 mM Tris/HCl (pH 8,8), 0,01% Tween 20 und 0,03 U/µl Taq DNA Polymerase (Biomaster) in die PCR eingesetzt. Die PCR wurde in einem Perkin Elmer 9600 Thermocycler mit dem nachfolgend aufgeführten Thermoprofil durchgeführt:

| | | |
|---|---|---|
| Initiale Denaturierung 35 Zyklen | 95 °C | 5 min |
| | 94 °C | 30 sec |
| | 57 °C | 30 sec |
| | 72 °C | 30 sec |
| | | |
| Finale Synthese | 72 °C | 5 min |

Nach Beendigung der PCR-Reaktion wurden die Amplifikationsprodukte mittels Agarose-Gelelektrophorese aufgetrennt und durch Anfärbung mit Ethidiumbromid visualisiert. Die erwarteten Produkte von 149 bp bzw. 151 bp Länge wurden nur in den Fällen beobachtet, in denen DNA von Stämmen der Spezies *L. monocytogenes* anwesend war. Die in den Gelen aufgetrennte DNA wurde mittels Standard-Methoden auf Nylon Filter transferiert und zur Überprüfung der Spezifität mit dem am 5'-Ende Digoxigenin-markierten Oligonukleotid LM 4 (Sequenz 4) hybridisiert. Die Hybridisierung erfolgte in 5 x SSC, 2 % Blocking Reagenz, 0,1 % Lauroylsarcosin, 0,02% SDS und 5 pmol/ml Sonde für 4 h bei 60 °C. Gewaschen wurde in 2 x SSC, 0,1 % SDS für 2 x 10 min bei 60 °C. Die Detektion erfolgte nach Standard-Methoden mittels Alkalischer Phosphatase Konjugate (Anti-Digoxiginin-AP Fab-Fragment, Fa. Boehringer Mannheim) in Anwesenheit von 5-Bromo-4-chloro-3-indolylphosphat und 4-Nitro-Blue Tetrazoliumchlorid (Fa. Boehringer Mannheim).
Auf den Filtern wurde nur in den Fällen eine Bande beobachtet; in denen zuvor eine Bande von 149 bp bzw. 151 bp auf dem Agarosegel sichtbar war. Somit wurde mittels PCR und Hybridisierung die Anwesenheit sämtlicher 103 getesteten *L. monocytogenes -* Stämme nachgewiesen. Hingegen wurde keiner der getesteten nicht zu dieser Spezies gehörenden Bakterienstämme mit diesem System erfaßt.

**Tabelle 1: Resultate der PCR-Amplifikation mit den Oligonukleotiden LM 1 und LM 2 (SEQ ID NO 1 und SEQ ID NO 2) bzw. LM 3 und LM 2 (SEQ ID NO 3 und SEQ ID NO 2) und jeweis nachfolgender Hybridisierung mit dem Oligonukleotid LM4 (SEQ ID NO 4)**

| **Spezies** | **Serovar** | **Stamm-bezeichnung** | **LM1/LM2** | **LM2/LM3** |
|---|---|---|---|---|
| *Listeria welshimeri* | | SLCC 767 | - | - |
| *Listeria welshimeri* | | SLCC 768 | - | - |
| *Listeria welshimeri* | | SLCC 5877 | - | - |
| *Listeria welshimeri* | | SLCC 5828 | - | - |
| *Listeria welshimeri* | | SLCC 6199 | - | - |
| *Listeria welshimeri* | | DSM 20650 | - | - |
| *Listeria seeligeri* | | SLCC 5921 | - | - |
| *Listeria seeligeri* | | SLCC 7303 | - | - |
| *Listeria seeligeri* | | SLCC 7309 | - | - |
| *Listeria seeligeri* | | SLCC 7329 | - | - |
| *Listeria seeligeri* | | SLCC 3954 | - | - |
| *Listeria seeligeri* | | DSM 20751 | - | - |
| *Listeria innocua* | | SLCC 5326 | - | - |
| *Listeria innocua* | | SLCC 7160 | - | - |
| *Listeria innocua* | | SLCC 7161 | - | - |
| *Listeria innocua* | | SLCC 7167 | - | - |
| *Listeria innocua* | | SLCC 7168 | - | - |
| *Listeria innocua* | | DSM 20649 | - | - |
| *Listeria innocua* | | SLCC 3408 | - | - |
| *Listeria innocua* | | NCTC 10528 | - | - |
| *Listeria innocua* | | SLCC 7139 | - | - |
| *Listeria grayi* | | DSM 20601 | - | - |
| *Listeria grayi* | | DSM 20596 | - | - |
| *Listeria grayi* | | BC 7308 | - | - |
| *Listeria ivanovii* | | DSM 20750 | - | - |
| *Listeria ivanovii* | | SLCC 2028 | - | - |
| *Listeria ivanovii* | | SLCC 2098 | - | - |
| *Listeria ivanovii* | | SLCC 2102 | - | - |
| *Listeria ivanovii* | | SLCC 2379 | - | - |
| *Listeria ivanovii* | | SLCC 4121 | - | - |
| *Listeria ivanovii* | | SLCC 4706 | - | - |
| *Listeria ivanovii* | | SLCC 4770 | - | - |
| *Listeria ivanovii* | | SLCC 5378 | - | - |
| *Listeria ivanovii* | | ATCC 19119 | - | - |
| *L. monocytogenes* | | ATCC 19111 | + | + |
| *L. monocytogenes* | | ATCC 19112 | + | + |
| *L. monocytogenes* | | ATCC 19113 | + | n.d. |
| *L. monocytogenes* | | ATCC 19114 | + | n.d. |
| *L. monocytogenes* | | ATCC 19115 | + | n.d. |
| *L. monocytogenes* | | ATCC 19116 | + | n.d. |
| *L. monocytogenes* | | ATCC 19117 | + | + |
| *L. monocytogenes* | | ATCC 19118 | + | n.d. |
| *L. monocytogenes* | | SLCC 53 | + | + |
| *L. monocytogenes* | | SLCC 2479 | + | + |
| *L. monocytogenes* | | SLCC 2482 | + | n.d. |
| *L. monocytogenes* | | SLCC 5835 | + | + |
| *L. monocytogenes* | 1 / 2 a | SLCC 4955 | + | + |
| *L. monocytogenes* | 1 / 2 a | SLCC 6204 | + | n.d. |
| *L. monocytogenes* | 1 / 2 a | SLCC 7149 | + | + |
| *L. monocytogenes* | 1 / 2 a | SLCC 7150 | + | + |
| *L. monocytogenes* | 1 / 2 a | SLCC 7153 | + | + |
| *L. monocytogenes* | 1 / 2 a | SLCC 7165 | + | n.d. |
| *L. monocytogenes* | 1 / 2 a | SLCC 7195 | + | n.d. |
| *L. monocytogenes* | 1 / 2 a | SLCC 7196 | + | n.d. |
| *L. monocytogenes* | 1 / 2 a | SLCC 7197 | + | n.d. |
| *L. monocytogenes* | 1 / 2 a | SLCC 7198 | + | n.d |
| *L*. *monocytogenes* | 1 / 2 a | SLCC 7973 | + | n.d. |
| *L. monocytogenes* | 1 / 2 a | SLCC 7053 | + | n.d. |
| *L. monocytogenes* | 1 / 2 a | SLCC 7054 | + | n.d. |
| *L*. *monocytogenes* | 1 / 2 a | SLCC 7055 | + | n.d. |
| *L. monocytogenes* | 1 / 2 b | SLCC 6031 | + | n.d. |
| *L*. *monocytogenes* | 1 / 2 b | SLCC 7163 | + | + |
| *L. monocytogenes* | 1 / 2 b | SLCC 7151 | + | + |
| *L. monocytogenes* | 1 / 2 b | SLCC 7152 | + | n.d. |
| *L*. *monocytogenes* | 1 / 2 b | SLCC 7354 | + | + |
| *L*. *monocytogenes* | 1 / 2 b | SLCC 7367 | + | n.d. |
| *L. monocytogenes* | 1 / 2 b | SLCC 7059 | + | n.d |
| *L. monocytogenes* | 1 / 2 c | SLCC 4950 | + | + |
| *L*. *monocytogenes* | 1 / 2 c | SLCC 6793 | + | + |
| *L. monocytogenes* | 1 / 2 c | SLCC 7154 | + | + |
| *L. monocytogenes* | 1 / 2 c | SLCC 7290 | + | n.d. |
| *L. monocytogenes* | 1 / 2 c | SLCC 7352 | + | n.d. |
| *L. monocytogenes* | 1 / 2 c | SLCC 7355 | + | n.d |
| *L. monocytogenes* | 3 a | SLCC 4949 | + | + |
| *L. monocytogenes* | 3 a | SLCC 7135 | + | n.d. |
| *L. monocytogenes* | 3 a | SLCC 7179 | + | n.d. |
| *L. monocytogenes* | 3b | SLCC 2540 | + | n.d. |
| *L. monocytogenes* | 3 b | SLCC 7140 | + | n.d. |
| *L. monocytogenes* | 3 b | SLCC 7381 | + | n.d |
| *L. monocytogenes* | 3 c | SLCC 2471 | + | + |
| *L. monocytogenes* | 4 a | SLCC 5069 | + | + |
| *L*. *monocytogenes* | 4 a | SLCC 5070 | + | n.d. |
| *L. monocytogenes* | 4 a / b | SLCC 7083 | + | n.d. |
| *L. monocytogenes* | 4 a / b | SLCC 7065 | + | n.d. |
| *L*. *monocytogenes* | 4 a / b | SLCC 7069 | + | + |
| *L. monocytoqenes* | 4 b | SLCC 4013 | + | + |
| *L. monocytogenes.* | 4 b | SLCC 7194 | + | + |
| *L*. *monocytogenes* | 4 b | SLCC 7356 | + | + |
| *L. monocytogenes* | 4 b | SLCC 7370 | + | + |
| *L. monocytogenes* | 4 b | SLCC 7372 | + | + |
| *L. monocytogenes* | 4 b | SLCC 7373 | + | n.d. |
| *L. monocytogences* | 4 b | SLCC 7374 | + | n.d. |
| *L. monocytogenes* | 4b | SLCC 788 | + | n.d. |
| *L. monocytogenes* | 4 b | SLCC 7056 | + | n.d. |
| *L. monocytogenes* | 4 b | SLCC 7057 | + | n.d. |
| *L. monocytogenes* | 4 b | SLCC 7058 | + | n.d. |
| *L. monocytogenes* | 4 b | SLCC 7060 | + | n.d. |
| *L. monocytogenes* | 4 b | SLCC 7061 | + | n.d. |
| *L. monocytogenes* | 4 b | SLCC 7062 | + | n.d. |
| *L. monocytogenes* | 4 b | SLCC 7063 | + | n.d. |
| *L. monocytogenes* | 4 b | SLCC 7064 | + | n.d. |
| *L. monocytogenes* | 4 b | SLCC 7066 | + | n.d. |
| *L. monocytogenes* | 4 b | SLCC 7067 | + | n.d. |
| *L. monocytogenes* | 4 b | SLCC 7068 | + | n.d. |
| *L. monocytogenes* | 4 b | SLCC 7069 | + | n.d. |
| *L. monocytogenes* | 4 b | SLCC 7070 | + | n.d. |
| *L. monocytogenes* | 4 b | SLCC 7071 | + | n.d. |
| *L. monocytogenes* | 4 b | SLCC 7072 | + | n.d. |
| *L. monocytogenes* | 4 b | SLCC 7073 | + | n.d. |
| *L. monocytogenes* | 4 b | SLCC 7074 | + | n.d. |
| *L. monocytogenes* | 4 b | SLCC 7075 | + | n.d. |
| *L. monocytogenes* | 4 b | SLCC 7076 | + | n.d. |
| *L. monocytogenes* | 4 b | SLCC 7077 | + | n.d. |
| *L. monocytogenes* | 4 b | SLCC 7078 | + | n.d. |
| *L. monocytogenes* | 4 b | SLCC 7079 | + | n.d. |
| *L. monocytogenes* | 4 b | SLCC 7080 | + | n.d. |
| *L. monocytogenes* | 4 b | SLCC 7081 | + | n.d. |
| *L. monocytogenes* | 4 b | SLCC 7082 | + | n.d. |
| *L. monocytogenes* | 4 b | SLCC 7084 | + | n.d. |
| *L. monocytogenes* | 4 b | SLCC 7085 | + | n.d. |
| *L. monocytogenes* | 4 b | SLCC.7086 | + | n.d. |
| *L. monocytogenes* | 4 b | SLCC 7087 | + | n.d. |
| *L. monocytogenes* | 4 b | SLCC 7008 | + | n. d. |
| *L. monocytogenes* | 4 b | SLCC 7089 | + | n. d. |
| *L. monocytogenes* | 4 b | SLCC 7090 | + | n.d. |
| *L. monocytogenes* | 4 b | SLCC 7091 | + | n.d. |
| *L. monocytogenes* | 4 b | SLCC 7092 | + | n.d. |
| *L. monocytogenes* | 4 c | SLCC 4925 | + | n.d. |
| *L. monocytogenes* | 4 c | SLCC 4954 | + | + |
| *L. monocytogenes* | 4 c | SLCC 6277 | + | + |
| *L. monocytogenes* | 4 c | SLCC 6813 | + | + |
| *L. monocytogenes* | 4 c | SLCC 6821 | + | + |
| *L. monocytogenes* | 4 c | SLCC 6823 | + | + |
| *L. monocytogenes* | 4 d | SLCC 2375 | + | n.d. |
| *L*. *monocytogenes* | 4 d | SLCC 4926 | + | + |
| *L. monocytogenes* | 4 d | SLCC 4952 | + | + |
| *L*. *monocytogenes* | 7 | SLCC 2622 | + | + |
| *Arthrobacter spec.* | | DSM 312 | - | n.d. |
| *Bacillus subtilis* | | ATCC 6051 | - | - |
| *Citrobacter freundii* | | DSM 30040 | - | - |
| *Citrobacter koseri* | | D5M 4595 | - | n.d. |
| *Clostridium bifermentans* | | DSM 630 | - | n.d. |
| *Clostridium sporogenes* | | IfGB 0303 | - | n.d. |
| *Enterobacter agglomerans* | | IfGB 0202 | - | n.d. |
| *Enterobacter cloacae* | | DSM 30054 | - | - |
| *Enterobacter gergoviae* | | BC 674 | - | n.d. |
| *Erwinia carotovora* | | DSM 30168 | - | n.d. |
| *Escherichia coli* | | DSM 30083 | - | n.d. |
| *Hafnia alvei* | | IfGB 0101 | - | n.d. |
| *Klebsiella oxytoca* | | DSM 5175 | - | n.d. |
| *Klebsiella pneumoniae* | | DSM 2026 | - | n.d. |
| *Lactobacillus spec.* | | IfGB 1401 | - | n.d. |
| *Lactob. bifermentans* | | BC 8463 | - | - |
| *Leuconostoc carnosum* | | DSM 5576 | - | n.d. |
| *Leucon. mesenteroides* | | DSM 2146 | - | n.d. |
| *Micrococcus citreus* | | IfGB 0601 | - | - |
| *Micrococcus luteus* | | DSM 348 | - | - |
| *Pediococcus damnosus* | | BC 505 | - | - |
| *Proteus mirabilis* | | IfGB 51 | - | - |
| *Proteus vulgaris* | | DSM 2041 | - | n.d. |
| *Pseudomonas aeruginosa* | | ATCC 10145 | - | n.d. |
| *Pseudomonas fluorescens* | | IfGB 0301 | - | - |
| *Salmonella spec.* | | BC 2426 | - | n.d. |
| *Salmonella typhimurium* | | BC 2157 | - | n.d. |
| *Serratia marcescens* | | BC 677 | - | - |
| *Shigella flexneri* | | DSM 4782 | - | n.d. |
| *Staphylococcus aureus* | | ATCC 6538 | - | - |
| *Streptococcus faecalis* | | DSM 20380 | - | n.d. |
| *Strept. faecalis* | | DSM 20478 | - | n.d. |
| *Strept. diacetylactis* | | BC 2149 | - | - |
| *Strept. thermophilus* | | DSM 20259 | - | n.d. |
| *Yersinia enterocolitica* | | DSM 4780 | - | n.d. |

| | | | | |
|---|---|---|---|---|
| IfGB: Institut für Gärungsgewerbe Berlin BC: BioteCon Stammsammlung SLCC: H.P.R. Seeliger Listeria Culture Collection, Würzburg ATCC: American Type Culture Collection, Rockville, USA DSM: Deutsche Sammlung von Microorganismen und Zellkulturen GmbH, Braunschweig n.d.: nicht durchgeführt | | | | |

### Beispiel 2: Online-Detektion von Bakterien der Spezies L. monocytogenes mit der Polymerase-Kettenreaktion

Aus Reinkulturen der in Tabelle 2 aufgeführten Stämme und Isolate wurde DNA mittels Standardverfahren isoliert. Je ca. 100 fg bis 100 ng dieser DNA-Präparationen wurden dann in Gegenwart von je 0,4 µM Oligonukleotid LM 1 und LM 2, je 0,2 µM LMF 1 (Markierung: 3'-Fluorescein), LMF 2 (Markierung: 3'-Fluorescein) und LMR 1 (Markierung: 5'-LC Red640 (Roche Diagnostics), 3'-Phosphat), 200 µM dNTP's (Roche Diagnostics), 4 mM MgCl₂, 3 µg/µl BSA (Roche Diagnostics), 16 mM (NH₄)₂SO₄, 67 mM Tris/HCl (pH 8,8), 0,01% Tween 20 und 0,04 U/µl Taq DNA Polymerase (HTB) in die PCR eingesetzt. Die PCR wurde in einem LightCycler der Firma Roche Diagnostics GmbH mit dem nachfolgend aufgeführten Thermoprofil durchgeführt:

| | | |
|---|---|---|
| Initiale Denaturierung | 95 °C | 2 min |
| 42 Zyklen | 97 °C | 0 sec |
| | 59 °C | 40 sec |

Während der PCR-Reaktion wurden Fluoreszenz-Signale (Detektions-Wellenlänge 640 nm) nur in den Fällen beobachtet, in denen DNA von Stämmen der Spezies *L. monocytogenes* anwesend war.

**Tabelle 2: Resultate der PCR-Amplifikation mit den Oligonukleotiden LM 1 und LM 2 (SEQ ID NO 1 und SEQ ID NO 2) und jeweis während der Amplifikationsreaktion erfolgenden Hybridisierung mit den Oligonukleotiden LMF1, LMF2 und LMR1 (SEQ ID NO 5, SEQ ID NO 6, SEQ ID NO 7 )**

| **Spezies** | **Serovar** | **Stamm-bezeichnung** | **LM1/LM2 LMF1/LMF2/LMR1** |
|---|---|---|---|
| *Bacillus staeroth.* | | DSM 456 | - |
| *Staphylococcus aureus* | | BC 197 | - |
| *Escherichia coli (VTEC)* | | BC 8318 | - |
| *Clostridium perfringens* | | BC 8799 | - |
| *Leuconostoc mesent.* | | DSM 20241 | - |
| *Listeria welshimeri* | | SLCC 767 | - |
| *Listeria welshimeri* | | SLCC 768 | - |
| *Listeria welshimeri* | | SLCC 5877 | - |
| *Listeria seeligeri* | | SLCC 5921 | - |
| *Listeria seeligeri* | | SLCC 7309 | - |
| *Listeria innocua* | | SLCC 5326 | - |
| *Listeria innocua* | | SLCC 7160 | - |
| *Listeria innocua* | | SLCC 7161 | - |
| *Listeria grayi* | | DSM 20601 | - |
| *Listeria ivanovii* | | SLCC 2028 | - |
| *Listeria ivanovii* | | SLCC 2098 | - |
| *Listeria ivanovii* | | SLCC 2102 | - |
| *L. monocytogenes* | 1 / 2 a | SLCC 4955 | + |
| *L. monocytogenes* | 1 / 2 a | SLCC 6204 | + |
| *L. monocytogenes* | 1 / 2 a | SLCC 7149 | + |
| *L. monocytogenes* | 1 / 2 a | SLCC 7150 | + |
| *L. monocytogenes* | 1 / 2 a | SLCC 7153 | + |
| *L. monocytogenes* | 1 / 2 b | SLCC 7151 | + |
| *L. monocytogenes* | 1 / 2 b | SLCC 7152 | + |
| *L. monocytogenes* | 1 / 2 b | SLCC 7354 | + |
| *L. monocytogenes* | 1 / 2 b | SLCC 7367 | + |
| *L. monocytogenes* | 1 / 2 b | SLCC 7059 | + |
| *L. monocytogenes.* | 1 / 2 c | SLCC 6793 | + |
| *L. monocytogenes* | 1 / 2 c | SLCC 7154 | + |
| *L. monocytogenes* | 1 / 2 c | SLCC 7290 | + |
| *L. monocytogenes* | 1 / 2 c | SLCC 7352 | + |
| *L. monocytogenes* | 1 / 2 c | SLCC 7355 | + |
| *L. monocytogenes* | 3 a | SLCC 4949 | + |
| *L. monocytogenes* | 3 a | SLCC 7135 | + |
| *L. monocytogenes* | 3 a | SLCC 7179 | + |
| *L. monocytogenes* | 3 b | SLCC 7140 | + |
| *L. monocytogenes* | 3 b | SLCC 7381 | + |
| *L. monocytogenes* | 3 c | SLCC 2471 | + |
| *L. monocytogenes* | 4 a | SLCC 5069 | + |
| *L. monocytogenes* | 4 a | SLCC 5070 | + |
| *L. monocytogenes* | 4 a / b | SLCC 7083 | + |
| *L. monocytogenes* | 4 a / b | SLCC 7065 | + |
| *L. monocytogenes* | 4 a / b | SLCC 7069 | + |
| *L. monocytogenes* | 4 b | SLCC 4013 | + |
| *L. monocytogenes* | 4 b | SLCC 7194 | + |
| *L. monocytogenes* | 4 b | SLCC 7356 | + |
| *L. monocytogenes* | 4 b | SLCC 7370 | + |
| *L. monocytogenes* | 4 b | SLCC 7372 | + |
| *L. monocytogenes* | 4 c | SLCC 4925 | + |
| *L. monocytogenes* | 4 c | SLCC 4.954 | + |
| *L. monocytogenes* | 4 c | SLCC 6277 | + |
| *L. monocytogenes* | 4 c | SLCC 6813 | + |
| *L. monocytogenes* | 4 c | SLCC 6821 | + |
| *L. monocytogenes* | 4 d | SLCC 2375 | + |
| *L. monocytogenes* | 4 d | SLCC 4926 | + |
| *L. monocytogenes* | 4 d | SLCC 4952 | + |
| *L. monocytogenes* | 7 | SLCC 2622 | + |

| | | | |
|---|---|---|---|
| IfGB: Institut für Gärungsgewerbe Berlin BC: BioteCon Stammsammlung SLCC: H.P.R. Seeliger Listeria Culture Collection, Würzburg ATCC: American Type Culture Collection, Rockville, USA DSM: Deutsche Sammlung von Microorganismen und Zellkulturen GmbH, Braunschweig | | | |

## Patentansprüche

1. Primer für eine PCR-Reaktion zum Nachweis von Bakterien der Spezies Listeria monocytogenes, wobei der Primer als Nukleinsäuremolekül einer Länge von 10 bis 30 Nukleotiden bezüglich mindestens 10 aufeinanderfolgenden Nukleotiden seiner Nukleotidkette
(i) mit 10 aufeinanderfolgenden Nukleotiden der Nukleinsäuremoleküle gemäß a) , b) , c) , d) , e) , f) oder g) identisch ist:
a) der SEQ ID NO 1 5'-GAA AAA GCA TTT GAA GCC AT-3' oder
b) der SEQ ID NO 2 5'-GCA ACT TCC GGC TCA GC-3' oder
c) der SEQ ID NO 3 5'-TCG AAA AAG CAT TTG AAG CC-3' oder
d) der SEQ ID NO 5 5'-CTI TTC ACA TGA GCT TCA CTC TGA CCR A-3'oder
e) der SEQ ID NO 6 5'-CTT TTT CTT TCA CTG GGT TTC CGA CAT-3' oder
f) der SEQ ID NO 7 5'-GAT GAT TTC TTT TTC TTT CAC TGG ATT TCC AAT AT-3' oder
g) der zu a), b), c), d), e) und f) jeweils komplementären Sequenz; oder
(ii) mit 9 von 10 aufeinanderfolgenden Nukleotiden der Nukleinsäuremoleküle gemäß (a) , (b) , (c) , (d) , (e) , (f) oder (g) übereinstimmt oder
(iii) mit 8 von 10 aufeinanderfolgenden Nukleotiden der Nukleinsäuremoleküle gemäß (a) , (b) , (c) , (d) , (e) , (f) oder (g) übereinstimmt oder
(iv) zu mindestens 90 % mit einem Nukleinsäuremolekül gemäß (a), (b), (c), (d), (e), (f) oder (g) homolog ist.

2. Primer nach Anspruch 1, ***gekennzeichnet* durch** eine Länge von 15 bis 30 Nukleotiden.

3. Primer
a) der SEQ ID NO 1 5'-GAA AAA GCA TTT GAA GCC AT-3' oder
b) der SEQ ID NO 2 5'-GCA ACT TCC GGC TCA GC-3' oder
c) der SEQ ID NO 3 5'-TCG AAA AAG CAT TTG AAG CC-3' oder
d) der SEQ ID NO 4 5'-GGT CAG AGT GAA GCT CAT GT-3' oder
e) der SEQ ID NO 5 5'-CTI TTC ACA TGA GCT TCA CTC TGA CCR A-3' oder
f) der SEQ ID NO 6 5'-CTT TTT CTT TCA CTG GGT TTC CGA CAT-3' oder
g) der SEQ ID NO 7 5'-GAT GAT TTC TTT TTC TTT CAC TGG ATT TCC AAT AT-3' oder
h) der zu a),b),c),d),e),f) und g) jeweils komplementären Sequenz.

4. Primer nach einem der vorhergehenden Ansprüche, **dadurch *gekennzeichnet,* daß** er einzelsträngig oder doppelsträngig vorliegt.

5. Primer nach einem der vorhergehenden Ansprüche, **dadurch *gekennzeichnet,* daß** er
(i) als DNA-Sequenz oder
(ii) als (i) entsprechende RNA-Sequenz oder
(iii) als PNA-Sequenz vorliegt,

6. Primer nach einem der vorhergehenden Ansprüche, **dadurch gekennzeichnet, daß** bis zu 20 % von mindestens 10 aufeinanderfolgenden Nukleotiden seiner Nukleotidkette, insbesondere 1 oder 2 Nukleotide, durch Nukleotide ersetzt sind, die bei Bakterien nicht natürlich vorkommen.

7. Primer nach einem der vorhergehenden Ansprüche, **dadurch gekennzeichnet, daß** der Primer dadurch oder zusätzlich dadurch modifiziert bzw. markiert ist, daß er eine oder mehrere radioaktive Gruppen, farbige Gruppen, fluoreszierende Gruppen, Gruppen zur Immobilisierung an fester Phase und/oder Gruppen für eine indirekte oder direkte Reaktion, insbesondere eine enzymatische Reaktion, insbesondere mit Hilfe von Antikörpern, Antigenen, Enzymen und/oder Substanzen mit Affinität zu Enzymen oder Enzymkomplexen, aufweist.

8. Kit zum Nachweis von Bakterien der Spezies *Listeria monocytogenes,* **gekennzeichnet durch** ein oder mehrere Primer gemäß einem der vorhergehenden Ansprüche.

9. Verwendung eines oder mehrerer Primer gemäß einem der Ansprüche 1 bis 7 oder eines Kits gemäß Anspruch 8 zum Nachweis der An- oder Abwesenheit von Bakterien der Spezies *Listeria monocytogenes.*

10. Verwendung nach Anspruch 9, **dadurch *gekennzeichnet,* daß** man eine Polymerase-Kettenreaktion für die Nukleinsäureamplifikation durchführt.

11. Verwendung nach Anspruch 10, **dadurch gekennzeichnet, dass** man die nachzuweisenden Bakterien von nicht-nachzuweisenden Bakterien anhand von Unterschieden der genomischen DNA und/oder RNA an an mindestens einer Nukleotidposition im Bereich eines der Nukleinsäuremoleküle gemäß Anspruch 3 unterscheidet.

12. Sonde zum Nachweis von Bakterien der Spezies Listeria monocytogenes, **dadurch gekennzeichnet, dass** die Sonde als Nukleinsäuremolekül eine der Sequenzen (a) bis (h) gemäß Anspruch 3 aufweist.

## Claims

1. A primer for a PCR-reaction for detecting bacteria of the species *Listeria monocytogenes,* wherein the primer is a nucleic acid molecule having a length of from 10 to 30 nucleotides, which with respect to at least 10 successive nucleotides of its nucleotide chain
(i) is identical to 10 successive nucleotides of the nucleic acid molecules according to a), b), c), d), e), f) or g)
a) of SEQ ID NO 1 5'-GAA AAA GCA TTT GAA GCC AT-3' or
b) of SEQ ID NO 2 5'-GCA ACT TCC GGC TCA GC-3' or
c) of SEQ ID NO 3 5'-TCG AAA AAG CAT TTG AAG CC-3' or
d) of SEQ ID NO 5 5'-CTI TTC ACA TGA GCT TCA CTC TGA CCR A-3' or
e) of SEQ ID NO 6 5'-CTT TTT CTT TCA CTG GGT TTC CGA CAT-3' or
f) of SEQ ID NO 7 5'-GAT GAT TTC TTT TTC TTT CAC TGG ATT TCC AAT AT-3' or
g) of the respective complementary sequence to a), b), c), d), e) and f); or
(ii) matches 9 out of 10 successive nucleotides of the nucleic acid molecules according to (a), (b), (c), (d), (e), (f) or (g); or
(iii) matches 8 out of 10 successive nucleotides of the nucleic acid molecules according to (a), (b), (c), (d), (e), (f) or (g); or
(iv) is at least 90% homologous to a nucleic acid molecule according to (a), (b), (c), (d), (e), (f) or (g).

2. Primer according to claim 1, **characterized by** a length of from 15 to 30 nucleotides.

3. Primer
a) of SEQ ID NO 1 5'-GAA AAA GCA TTT GAA GCC AT-3' or
b) of SEQ ID NO 2 5'-GCA ACT TCC GGC TCA GC-3' or
c) of SEQ ID NO 3 5'-TCG AAA AAG CAT TTG AAG CC-3' or
d) of SEQ ID NO 4 5'-GGT CAG AGT GAA GCT CAT GT-3' or
e) of SEQ ID NO 5 5'-CTI TTC ACA TGA GCT TCA CTC TGA CCR A-3' or
f) of SEQ ID NO 6 5'-CTT TTT CTT TCA CTG GGT TTC CGA CAT-3' or
g) of SEQ ID NO 7 5'-GAT GAT TTC TTT TTC TTT CAC TGG ATT TCC AAT AT-3'or
h) of the respective complementary sequence to a), b), c), d), e), f) and g).

4. Primer according to any of the preceding claims, **characterized in that** it is present in single-stranded or double-stranded form.

5. Primer according to any of the preceding claims, **characterized in that** it is present
(i) as DNA sequence or
(ii) as RNA sequence corresponding to (i) or
(iii) as PNA sequence.

6. Primer according to any of the preceding claims, **characterized in that** up to 20% of at least 10 successive nucleotides of its nucleotide chain, in particular 1 or 2 nucleotides, have been replaced by nucleotides not naturally present in bacteria.

7. Primer according to any of the preceding claims, **characterized in that** the primer has been modified or labeled by or additionally by having one or more radioactive groups, colored groups, fluorescent groups, groups for immobilization on a solid phase and/or groups for an indirect or direct reaction, in particular for an enzymatic reaction, in particular with the aid of antibodies, antigens, enzymes and/or substances with affinity to enzymes or enzyme complexes.

8. Kit for detecting bacteria of the species *Listeria monocytogenes,* **characterized by** one or more primer(s) according to any of the preceding claims.

9. Use of one or more primer(s) according to any of claims 1 to 7 or of a kit according to claim 8 for detecting the presence or absence of bacteria of the species *Listeria monocytogenes.*

10. Use according to claim 9, **characterized in that** for the nucleic acid amplification a polymerase chain reaction is carried out.

11. Use according to claim 10, **characterized in that** the bacteria to be detected are distinguished from the bacteria not to be detected on the basis of differences in the genomic DNA and/or RNA in at least one nucleotide position in the region of one of the nucleic acid molecules according to claim 3.

12. Probe for detecting bacteria of the species *Listeria monocytogenes,* **characterized in that** the probe is a nucleic acid molecule corresponding to one of the sequences (a) to (h) according to claim 3.

## Revendications

1. Amorce pour une réaction de PCR pour détecter des bactéries de l'espère *Listeria monocytogenes,* sachant que l'amorce en tant que molécule d'acide nucléique d'une longueur de 10 à 30 nucléotides est identique au niveau d'au moins 10 nucléotides consécutifs de sa chaîne de nucléotides
(i) à 10 nucléotides consécutifs de la molécule d'acide nucléique selon a), b), c), d), e), f) ou g) :
a) de SEQ ID N° 1 : 5'-GAA AAA GCA TTT GAA GCC AT-3' ou
b) de SEQ ID N° 2 : 5' -GCA ACT TCC GGC TCA GC-3' ou
c) de SEQ ID N° 3 : 5'-TCG AAA AAG CAT TTG AAG CC-3' ou
d) de SEQ ID N° 5 : 5'-CTI TTC ACA TGA GCT TCA CTC TGA CCR A-3' ou
e) de SEQ ID N° 6 : 5'-CTT TTT CTT TCA CTG GGT TTC CGA CAT-3' ou
f) de SEQ ID N° 7 : 5'-GAT GAT TTC TTT TTC TTT CAC TGG ATT TCC AAT AT-3' ou
g) de la séquence complémentaire respectivement aux séquences a), b), c), d), e) et f) ; ou
(ii) est conforme à 9 nucléotides consécutifs sur 10 des molecules d'acide nucléique selon (a), (b), (c), (d), (e), (f) ou (g) ou
(iii) est conforme à 8 nucléotides consécutifs sur 10 des molécules d'acide nucléique selon (a), (b), (c), (d), (e), (f) ou (g) ou
(iv) est homologue à au moins 90 % d'une molécule d'acide nucléique selon (a), (b), (c), (d), (e), (f) ou (g).

2. Amorce selon la revendication 1, **caractérisée par** une longueur de 15 à 30 nucléotides.

3. Amorce
a) de SEQ ID N° 1 : 5'-GAA AAA GCA TTT GAA GCC AT-3' ou
b) de SEQ ID N° 2 : 5'-GCA ACT TCC GGC TCA GC-3' ou
c) de SEQ ID N° 3: 5'-TCG AAA AAG CAT TTG AAG CC-3' ou
d) de SEQ ID N° 4: 5'-GGT CAG AGT GAA GCT CAT GT-3' ou
e) de SEQ ID N° 5: 5'-CTI TTC ACA TGA GCT TCA CTC TGA CCR A-3' ou
f) de SEQ ID N° 6 : 5'-CTT TTT CTT TCA CTG GGT TTC CGA CAT-3' ou
g) de SEQ ID N° 7 : 5'-GAT GAT TTC TTT TTC TTT CAC TGG ATT TCC AAT AT-3' ou
h) de la séquence complémentaire respectivement aux séquences a), b), c), d), e), f) et g).

4. Amorce selon l'une quelconque des revendications précédentes, **caractérisée en ce qu'**elle se présente sous une forme à brin simple ou à brin double.

5. Amorce selon l'une quelconque des revendications précédentes, **caractérisée en ce qu'**elle se présente sous la forme
(i) d'une séquence d'ADN
(ii) de la séquence d'ARN correspondant à la séquence (i) ou
(iii) de la séquence d'ANP.

6. Amorce selon l'une quelconque des revendications précédentes, **caractérisée en ce que** jusqu'à 20 % d'au moins 10 nucléotides consécutifs de sa chaîne de nucléotides, en particulier 1 ou 2 nucléotides, sont remplacés par des nucléotides qui ne sont pas présents à l'état naturel dans les bactéries.

7. Amorce selon l'une quelconque des revendications précédentes, **caractérisée en ce que** l'amorce est modifiée ou marquée, ou est de plus modifiée et marquée, **en ce qu'**elle présente un ou plusieurs groupes radioactifs, groupes colorés, groupes fluorescents, groupes d'immobilisation sur une phase solide et/ou groupes de réaction indirecte ou directe, en particulier, une réaction enzymatique, en particulier à l'aide d'anticorps, d'antigènes, d'enzymes et/ou de substance présentant une affinité avec les enzymes ou les complexes enzymatiques.

8. Kit de détection de bactéries de l'espèce *Listeria monocytogenes,* **caractérisé par** une ou plusieurs amorces selon l'une quelconque des revendications précédentes.

9. Utilisation d'une ou de plusieurs amorces selon l'une des revendications 1 à 7 ou d'un kit selon la revendication 8 pour détecter la présence ou l'absence de bactéries de l'espèce *Listeria monocytogenes.*

10. Utilisation selon la revendication 9, **caractérisée en ce que** l'on réalise une réaction en chaîne polymérase pour l'amplification d'acide nucléique.

11. Utilisation selon la revendication 10, **caractérisée en ce que** l'on distingue les bactéries à détecter des bactéries qui ne sont pas à détecter sur la base de différences de l'ADN génomique et/ou de l'ARN sur au moins une position de nucléotide dans la région d'une molécule d'acide nucléique selon la revendication 3.

12. Sonde pour la détection de bactéries de l'espèce *Listeria monocytogenes,* **caractérisée en ce que** la sonde, en tant que molécule d'acide nucléique, présente l'une des séquences de (a) à (h) selon la revendication 3.


### SEQUENZPROTOKOLL

<110> Biotecon Gesellschaft für Biotechnologische Entwic
   Scheu, Pia
   Gasch, Alerander
   Berghof, Kornelia
<120> Oligonukleotide, Verfahren und Kit zur Detektion von Listeria monocytogenes durch Nukleinsäureamplifikation und/oder -hybridisierung
<130> PCT/BP 99/06453
<140> PCT/BP 99/06953
   <141> 1999-09-02
<150> DE 198 40 044-6
   <151> 1998-09-02
<160> 7
<170> PatentIn Ver. 2.1
<210> 1
   <211> 20
   <212> DNA
   <213> Listeria monocytogenes
<400> 1
   gaaaaagcat ttgaagccat 20
<210> 2
   <211> 17
   <212> DNA
   <213> Listeria monocytogenes
<400> 2
   gcaacttccg gctcagc 17
<210> 3
   <211> 20
   <212> DNA
   <213> Listeria monocytogenes
<400> 3
   tcgaaaaagc atttgaagcc 20
<210> 4
   <211> 20
   <212> DNA
   <213> Listeria monocytogenes
<400> 4
   ggtcagagtg aagctcatgt 20
<210> 5
   <211> 28
   <212> DNA
   <213> Listeria monocytogenes
<220>
   <221> misc_difference
   <222> (3)
   <223> N=i
<400> 5
   ctnttcacat gagcttcact ctgaccra 28
<210> 6
   <211> 27
   <212> DNA
   <213> Listeria monocytogenes
<400> 6
   ctttttcttt cactgggttt ccgacat 27
<210> 7
   <211> 35
   <212> DNA
   <213> Listeria monocytogenes
<400> 7
   gatgatttct ttttctttca ctggatttcc aatat 35